# EUROPÄISCHE PATENTSCHRIFT

(11) **EP 0 378 183 B1**
(45) Veröffentlichungstag und Bekanntmachung des Hinweises auf die Patenterteilung: **02.11.1994**
(21) Anmeldenummer: 90100395.4
(22) Anmeldetag: 09.01.1990
(51) Int. Cl.: A61B 19/02, B65D 81/32, B65B 9/08, B65B 9/04

(54) **Sterile Kunststoffpackung**
Sterile plastic package
Emballage stérile en matière plastique

(30) Priorität: 12.01.1989 DE 3900702
(43) Veröffentlichungstag der Anmeldung: 18.07.1990
(73) Patentinhaber: Karl H. Sengewald GmbH & Co. KG, D-33790 Halle (DE)
(72) Erfinder: Strassmann, Günter, D-4800 Bielefeld 1 (DE); Sengewald, Karl H., Dr., D-4802 Halle/Westfalen 1 (DE)
(74) Vertreter: Selting, Günther, Dipl.-Ing.

(56) Entgegenhaltungen:
- WO-A-84/02892
- BE-A- 894 377
- DE-A- 3 426 465
- DE-C- 544 820
- DE-U- 8 530 365
- FR-A- 2 423 413
- FR-E- 75 473
- GB-A- 746 325
- US-A- 3 319 538
- US-A- 3 345 988
- US-A- 3 648 704
- US-A- 4 372 098
- US-A- 4 395 254

## Beschreibung

Die Erfindung betrifft eine sterile Kunststoffpackung der im Oberbegriff des Patentanspruchs 1 angegebenen Art.

Medizinische Geräte, wie Spritzen, Kanülen, Katheter oder auch Produkte wie Verbandstoffe und Einmalhandschuhe, müssen steril verpackt werden, wobei die Packung einerseits eine Sterilisation des verpackten Produktes ermöglicht und andererseits Rekontaminationen im verpackten Zustand verhindert. Üblicherweise werden für sterile Produkte Kunststoffpackungen benutzt, die eine gas- oder dampfdurchlässige Wand aufweisen. Nach dem Einfüllen der Produkte in die Packungen und nach dem Verschließen der Packungen werden Packungen und Produkte in einer Sterilisationskammer sterilisiert. Für die Sterilisierung ist ein beträchtlicher Aufwand an Apparaturen und Zeit erforderlich. Außerdem können bei langer Lagerung unter ungünstigen Bedingungen Rekontaminationen des verpackten Gutes eintreten.

Eine aus DE-U-85 30 365 bekannte Kunststoffpackung besteht aus zwei an ihren Rändern miteinander verschweißten Folien, die zwei Aufnahmeräume bilden. In dem einen Aufnahmeraum ist eine sterilisierte Spritze enthalten und der andere Aufnahmeraum enthält einen faserigen absorbierenden Bausch, der mit einem Desinfektionsmittel imprägniert ist. Die Packung dient dazu, dem Arzt das für eine Punktion des Patienten erforderliche Material in einer selbständigen Einheit zur Verfügung zu stellen, so daß dieses Material nicht in unterschiedlichen Verpackungen mitgeführt werden muß. Die Desinfizierung der Spritze mit dem Bausch erfolgt, nachdem beide Aufnahmeräume nach außen geöffnet und die darin enthaltenen Gegenstände entnommen wurden.

Für die Herstellung von Mehrkomponenten-Gemischen, deren Komponenten erst kurz vor Gebrauch gemischt werden dürfen, sind aus DE 34 26 465 A1 und CH 398 453 Kunststoffpackungen bekannt, die zwei voneinander getrennte Kammern haben, welche durch eine Sollbruchstelle voneinander getrennt sind. Die Sollbruchstelle kann entweder durch Drücken auf eine der Kammern oder durch Ziehen an einem Aufreißorgan gesprengt werden, woraufhin die in einer der Kammer enthaltene Substanz in die andere Kammer hinein ausgequetscht werden und beide Substanzen miteinander vermischt werden können. Solche Mehrkomponenten-Packungen sind für miteinander reagierende Substanzen, z.B. Grundsubstanz und Härtemittel, bestimmt, um Kleber oder Zemente zu bilden.

Aus DE-B 1 274 952 ist eine Beutelherstellungsmaschine für Muldenbeutel bekannt, bei der eine endlose Reihe aus zwei nebeneinander angeordneten Muldenbeuteln hergestellt wird, deren Mulden an einer Fülleinrichtung mit demselben Material gefüllt und anschließend mit einer Deckfolie verschlossen werden.

Der Erfindung liegt die Aufgabe zugrunde, eine sterile Kunststoffpackung zu schaffen, die nach ihrer Herstellung keine Sterilisationsbehandlung im Sterilisationsraum erfordert und bei der der Packungsinhalt jederzeit auf einfache Weise, ggf. vom Benutzer, sterilisiert werden kann.

Die Lösung dieser Aufgabe erfolgt erfindungsgemäß mit den im kennzeichnenden Teil des Patentanspruchs 1 angegebenen Merkmalen.

Die erfindungsgemäße Kunststoffpackung weist außer dem Aufnahmeraum, der das verpackte Gut enthält, eine mit Sterilisationsmittel gefüllte Hilfskammer auf. Aufnahmeraum und Hilfskammer sind durch eine Sollbruchstelle voneinander getrennt. Durch Zerbrechen der Sollbruchstelle wird die Hilfskammer mit dem Aufnahmeraum verbunden, während das gesamte System nach außen hin geschlossen bleibt. Dabei tritt Sterilisationsmittel in den Aufnahmeraum ein und benetzt den verpackten Gegenstand und tötet dort eventuell vorhandene Mikroorganismen. Ein wesentlicher Vorteil der Erfindung besteht darin, daß die Kunststoffpackung ein nach außen kontaminationsdicht abgeschlossenes System bildet, dessen Wände gasdicht abschließen können. Zur Durchführung der Sterilisation wird dieses geschlossene System nach außen nicht geöffnet, sondern es entsteht lediglich eine Verbindung der beiden Kammern durch die zerbrochene Sollbruchstelle hindurch. Die Sterilisierung kann jederzeit erfolgen, also auch kurze Zeit vor dem Öffnen der Packung und der Benutzung des Packungsinhalts. Das Zerbrechen der Sollbruchstelle kann dadurch erfolgen, daß ein Druck auf die mit flüssigem Sterilisationsmittel gefüllte Hilfskammer ausgeübt wird. Das Sterilisationsmittel fließt dann unter dem von außen aufgebrachten Druck in den Aufnahmeraum.

Zum besseren Verteilen des Sterilisationsmittels im Aufnahmeraum kann dort eine Saugeinlage vorgesehen sein. Diese Saugeinlage aus einem hydrophilen Fasermaterial wird mit dem Sterilisationsmittel durchtränkt und bewirkt einerseits dessen Verteilung, verhindert andererseits aber auch die Bildung zu starker örtlicher Flüssigkeitsansammlungen an dem verpackten Gut. Die Saugeinlage kann als Beutel ausgebildet sein, in dem sich das verpackte Gut befindet. Bei dem verpackten Gut kann es sich um medizinische Geräte, Hilfsprodukte oder andere Steril-Produkte handeln.

Zur Vermeidung von Verschiebungen der Saugeinlage in dem Aufnahmeraum ist die Saugeinlage zweckmäßigerweise an mindestens einer Wand des Aufnahmeraums fixiert. Die Saugeinlage kann auch ein Beutel sein, in den das Packungsgut eingesetzt wird und der einen Innenbeutel des Aufnahmeraums bildet.

Zweckmäßigerweise ist im Bereich der Sollbruchstelle ein Peel-Streifen zwischen Unterfolie und Oberfolie eingeschweißt. Ein solcher Peel-Streifen bewirkt eine leicht aufreißbare Verbindung zwischen Oberfolie und Unterfolie, wobei die Festigkeit dieser Verbindung geringer ist als eine direkte Schweißverbindung zwischen den beiden genannten Folien. Entsprechende Peel-Streifen sind in der Kunststoff-Technik bekannt.

Die erfindungsgemäße Kunststoffpackung kann sowohl als Flachbeutel als auch als Muldenbeutel ausgeführt sein. Bei einem Flachbeutel bestehen die Beutelwandungen aus relativ flexiblen flachen Folien, während ein Muldenbeutel eine tiefgezogene gemuldete Unterfolie aufweist, die mit einer flexiblen Oberfolie bedeckt ist.

Die Erfindung betrifft ferner Vorrichtungen zur Herstellung der Kunststoffpackung.

Im folgenden werden unter Bezugnahme auf die Zeichnungen Ausführungsbeispiele der Erfindung näher erläutert.

Es zeigen:
- Fig. 1: ein Ausführungsbeispiel einer Kunststoffpackung als Flachbeutel in Draufsicht,
- Fig. 2: einen Schnitt entlang der Linie II-II von Fig. 1,
- Fig. 3: eine Vorrichtung zur Herstellung der Flachbeutel nach Fign. 1 und 2,
- Fig. 4: einen schematischen Längsschnitt durch einen Muldenbeutel, und
- Fig. 5: eine schematische Darstellung einer Vorrichtung zur Herstellung der Muldenbeutel nach Fig. 4.

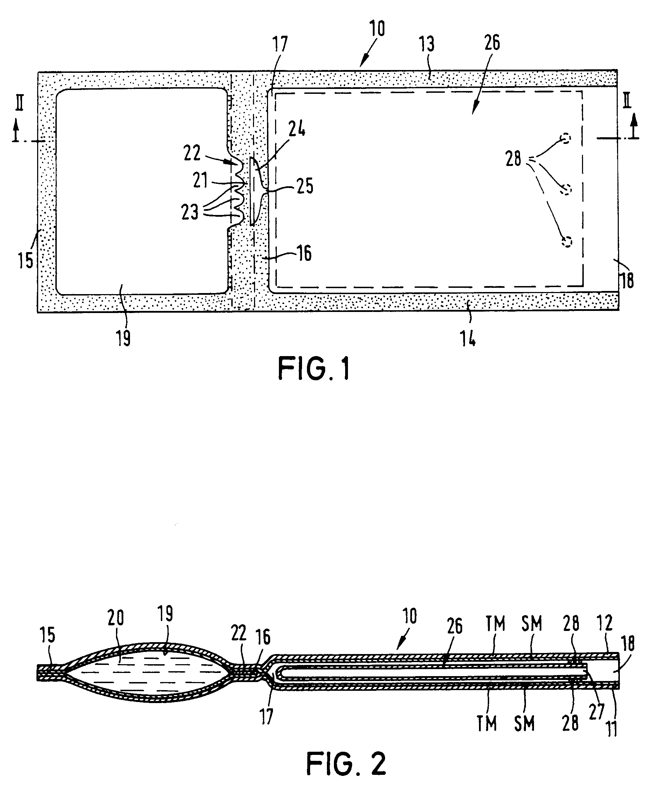

Die in den Fign 1 und 2 dargestellte Kunststoffpackung besteht aus einem Flachbeutel 10, aus einer Unterfolie 11 und einer Oberfolie 12. Die beiden Folien 11,12 bestehen jeweils aus einem Trägermaterial TM von relativ hoher Festigkeit und relativ geringer Gasdurchlässigkeit, wie z.B. PET, PA, OPP, und einem Siegelmaterial SM, das an der Packungsinnenseite angeordnet ist und durch Wärmebehandlung siegelungsfähig ist, z.B. aus PE oder PP.

Unterfolie 11 und Oberfolie 12 sind durch Längsschweißnähte 13,14 miteinander verbunden. Zwischen den Längsschweißnähten 13,14 erstrecken sich zwei Querschweißnähte 15 und 16.

Der Aufnahmeraum 17 für die zu verpackenden Gegenstände wird durch die Längsschweißnähte 13,14 und durch die Querschweißnaht 16 begrenzt. Dieser Aufnahmeraum 17 ist an dem einen Ende 18 zunächst offen, damit das Packungsgut eingefüllt werden kann. Dieses Ende 18 wird später mit einer weiteren Querschweißnaht verschlossen.

Die Längsschweißnähte 13 und 14 begrenzen zusammen mit den Querschweißnähten 15 und 16 eine Hilfskammer 19, die mit flüssigem Sterilisationsmittel 20 gefüllt ist. Bei dem Sterilisationsmittel handelt es sich beispielsweise um das Produkt Sporicidin der Firma Sporicidin International, einem chemischen Sterilisationsprodukt, das Mikroorganismen bei Raumtemperatur innerhalb von ein bis zwei Minuten abtötet.

In der Querschweißnaht 16, die die Hilfskammer 19 von dem Aufnahmeraum 17 trennt, ist eine Sollbruchstelle 21 vorgesehen. Hierzu ist zwischen Unterfolie 11 und Oberfolie 12 ein Peel-Streifen 22 eingelegt, der quer zur Packung verläuft und - mit Ausnahme seines mittleren Bereiches - vollständig von der Querschweißnaht 16 überdeckt wird. Im mittleren Bereich ist die Siegelkontur der Querschweißnaht 16 so ausgebildet, daß mehrere ungesiegelte Vorsprünge 23 gebildet werden, die sich von der Hilfskammer 19 in Richtung auf den Aufnahmeraum 17 erstrecken und die einen Teil der Breite des Peel-Streifens 22 überdecken. Von dem Aufnahmeraum 17 aus erstreckt sich ein trichterförmig erweiterter ungesiegelter Bereich 24 in Richtung auf die Hilfskammer 19. Zwischen den ungesiegelten Vorsprüngen 23 und dem ungesiegelten Bereich 24, die einen geringen Abstand voneinander haben, erstreckt die Sollbruchstelle 21, die den Peel-Streifen 22 überdeckt. Dies bedeutet, daß sowohl die Vorsprünge 23 als auch der Bereich 24 jeweils einen Rand des Peel-Streifens 22 überlappen.

Wenn auf die prall mit Flüssigkeit gefüllte Hilfskammer 19 ein Druck ausgeübt wird, wird die von der Sollbruchstelle 21 auf dem Peel-Streifen 22 gebildete Brücke von diesem Flüssigkeitsdruck gesprengt und Sterilisationsmittel 20 gelangt in den trichterförmigen Bereich 24 und von dort durch die Verengung 25 in den Aufnahmeraum 17.

Aus vorstehendem ergibt sich, daß die Sollbruchstelle 21 durch zwei Maßnahmen gebildet wird, nämlich einerseits durch das Einbetten des Peel-Streifens in die Querschweißnaht 16 und zum anderen dadurch, daß in der Mitte der Breite der Packung die Schweißkontur derart ausgebildet ist, daß die Schweißnaht 16 sich örtlich nur über einen geringen Teil der Breite des Peel-Streifens erstreckt, während die ungeschweißten Vorsprünge 23 und der ungeschweißte Bereich 24 von beiden Seiten her über die Ränder des Peel-Streifens ragen und direkte Verbindung mit der Hilfskammer 19 bzw. dem Aufnahmeraum 17 haben.

Zur besseren Verteilung des Sterilisationsmittels im Aufnahmeraum 17 enthält der Aufnahmeraum eine Saugeinlage 26 aus einem hydrophilen Fasermaterial, z.B. aus Vliesstoff. Die Saugeinlage 26 ist bei dem vorliegenden Ausführungsbeispiel als rechteckiger flacher Beutel ausgebildet, der aus zwei an seinen Rändern miteinander verschweißten Bahnen besteht, wobei die Beutelöffnung 27 der Hilfskammer 19 abgewandt ist. Zur Lagesicherung der Saugeinlage 26 im Aufnahmeraum 17 ist die obere Wand des Beutels 26 über Siegelungspunkte 28 an die Oberfolie 12 angesiegelt. Entsprechende Siegelungspunkte verbinden in der Nähe der Öffnung 27 die untere Wand der Saugeinlage mit der Unterfolie 11. Auf diese Weise wird beim Öffnen der Beutelöffnung 18 zugleich die Öffnung 27 der Saugeinlage 26 geöffnet, damit das Packungsgut eingelegt werden kann. Das für die Saugeinlage 26 verwendete Kunststoffmaterial sollte eine höhere Schmelztemperatur haben als das für den Folienbeutel verwendete Siegelmaterial, um zu gewährleisten, daß das Material der Saugeinlage bei der Befestigung an der Beutelfolie nicht mit sich selbst versiegelt, wodurch der innere Saugbeutel verschlossen würde.

Die Saugeinlage 26 endet im Abstand von der Beutelöffnung 18, so daß genügend Platz zur Verfügung steht, um die Beutelöffnung 18 mit einer weiteren Querschweißnaht zu verschließen.

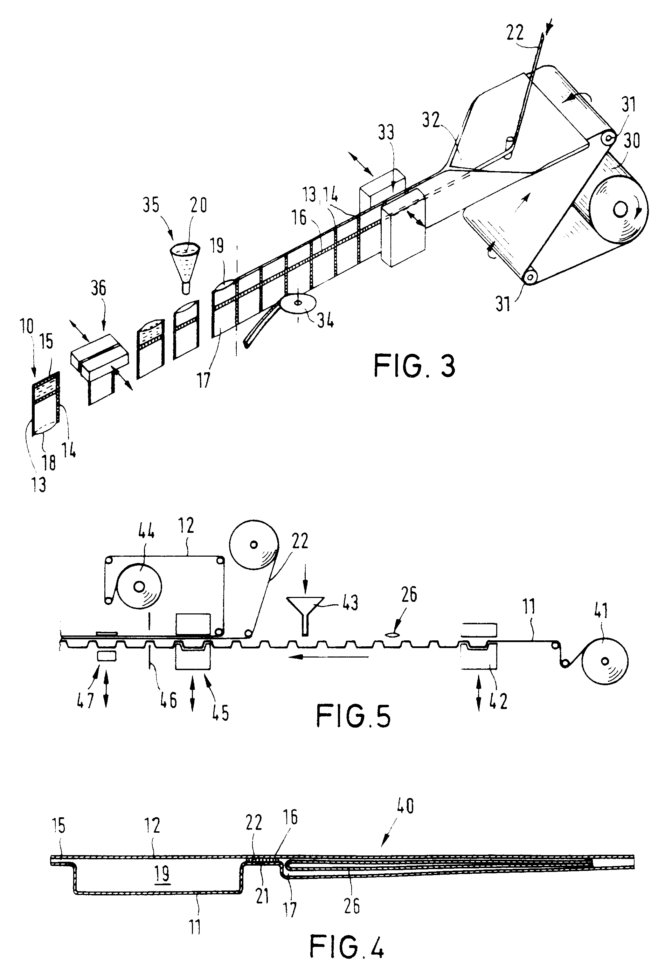

Fig. 3 zeigt schematisch eine Vorrichtung zur Herstellung des in den Fign. 1 und 2 dargestellten Beutels. Gemäß Fig. 3 wird eine Folienbahn von einer Vorratstrommel 30 abgezogen und über Umlenkwalzen 31 zu einer Faltvorrichtung 32 geführt, die die Folienbahn umfaltet und zu einem einseitig offenen Halbschlauch zusammenlegt. In diesen Halbschlauch wird der Peel-Streifen 22 eingeführt. Die beiden zusammenliegenden Wände des Halbschlauchs werden an der ersten Siegelstation 33 miteinander versiegelt, wobei die beiden Längsschweißnähte 13,14 und die Querschweißnaht 16 erzeugt werden. Die Flachbeutel sind dabei quer zur Längsrichtung des Folienhalbschlauchs orientiert. Die Faltlinie befindet sich am unteren Ende des Aufnahmeraums 17. Durch eine Trennvorrichtung 34 wird der Bereich der Faltlinie abgetrennt, so daß die Beutelöffnung 18 entsteht. An einer Füllstation 35 wird Sterilisationsmittel 20 in die oben offenen Hilfskammern 15 eingefüllt und diese werden anschließend in einer zweiten Siegelstation 36 verschlossen, in der die Querschweißnaht 15 erzeugt wird. Der Beutel ist dann für die Aufnahme des zu verpackenden Gegenstandes vorbereitet. Die Hilfskammer 19 ist geschlossen und enthält das Sterilisationsmittel 20. An geeigneter Stelle des Beutelherstellungsprozesses kann das Abtrennen der einzelnen Flachbeutel 10 von dem Folienband erfolgen. Die Saugeinlage 26 wird von Hand oder automatisch in den Aufnahmeraum 17 der Beutel eingebracht. Anstelle des Ansiegelns der Saugeinlage an die Beutelwände kann auch ein Anheften mit einem bei niedriger Temperatur siegelnden Siegellack oder mit Kaltsiegellack erfolgen. Ebenso können Kleber oder Siegellacke punkt- oder streifenförmig auf die Folie aufgebracht werden. Es ist auch möglich, anstelle einer beutelförmigen Saugeinlage eine einfachliegende Saugbahn zu verwenden.

Fig. 4 zeigt einen Muldenbeutel, bei dem die Unterfolie 11 aus einem durch Tiefziehen gemuldeten Folienmaterial besteht, während die Oberfolie 12 flach auf die flanschartigen Ränder der Unterfolie 11 aufgelegt und mit diesen verschweißt ist. Auch bei dem Muldenbeutel 40 ist eine mittlere Querschweißnaht 16 mit eingebettetem Peel-Streifen 22 zur Bildung der Sollbruchstelle 21 vorgesehen. Die Querschweißnaht 16 trennt die Hilfskammer 19 vom Aufnahmeraum 17, der eine Saugeinlage 26 enthält.

Die Vorrichtung zur Herstellung der Muldenbeutel 40 von Fig. 4 weist eine Vorratsrolle 41 auf, von der die Unterfolie 11 abgezogen wird. In einer Formstation 42 werden in der Unterfolie 11 die beiden Mulden zur Bildung des Aufnahmeraums 17 und der Hilfskammer 19 geprägt, wobei in Fig. 5 jeweils nur die Mulde des Aufnahmeraums 17 sichtbar ist. Nach dem Ausformen der Mulden wird die Saugeinlage 26 in jeden Aufnahmeraum gelegt. An einer Füllstation 43 wird Sterilisationsmittel 20 in die Hilfskammer 19 eingefüllt. Im Anschluß hieran wird der Peel-Streifen 22 auf den Steg zwischen Aufnahmeraum 17 und Hilfskammer 19 aufgelegt und schließlich wird die Oberfolie 12 zugeführt, die von einer Vorratsrolle 44 abgezogen wird. An der Siegelstation 45 wird die Oberfolie 12 mit der Unterfolie 11 verbunden. Danach folgt an einer Trennstation 46 das Abtrennen der einzelnen Muldenbeutel und schließlich wird in einer weiteren Siegelstation 47 die Saugeinlage 26 in der schon beschriebenen Weise an der Unterfolie 11 bzw. der Oberfolie 12 mit Siegelpunkten 28 fixiert.

Das Einlegen der Gegenstände kann dadurch erfolgen, daß diese Gegenstände bereits in der Saugeinlage 26 enthalten sind, wenn diese in den Aufnahmeraum gelegt wird, oder auch erst im Anschluß an die Siegelstation 47, wenn an der Siegelstation 45 noch eine Beutelöffnung offengelassen wird.

## Patentansprüche

1. Sterile Kunststoffpackung mit einer Unterfolie (11) und einer Oberfolie (12), die einen ein medizinisches Gerät enthaltenden Aufnahmeraum (17) begrenzen und an mindestens einem Ende durch eine Querschweißnaht verbunden sind,
**dadurch gekennzeichnet,**
daß sich an den Aufnahmeraum (17) mindestens eine Hilfskammer (19) anschließt, die ein Sterilisationsmittel (20) enthält, und daß zwischen der Hilfskammer (19) und dem Aufnahmeraum (17) eine manuell aufreißbare Sollbruchstelle (21) vorgesehen ist, die zerbrochen werden kann, um bei Aufrechterhaltung der Abdichtung nach außen einen Durchlaß von der Hilfskammer (19) in den Aufnahmeraum (17) freizugeben.

2. Kunststoffpackung nach Anspruch 1, dadurch gekennzeichnet, daß der Aufnahmeraum (17) eine Saugeinlage (26) zum Verteilen des Sterilisationsmittels enthält.

3. Kunststoffpackung nach Anspruch 2, dadurch gekennzeichnet, daß die Saugeinlage an der Oberfolie (12) und/oder der Unterfolie (11) fixiert ist.

4. Kunststoffpackung nach Anspruch 2 oder 3, dadurch gekennzeichnet, daß die Saugeinlage (26) ein Beutel ist, dessen Öffnung (27) sich an dem der Hilfskammer (19) abgewandten Ende befindet.

5. Kunststoffpackung nach Anspruch 4, dadurch gekennzeichnet, daß der eine Rand der Beutelöffnung (27) mit der Oberfolie (12) und der andere mit der Unterfolie (11) verbunden ist.

6. Kunststoffpackung nach einem der Ansprüche 1 bis 5, dadurch gekennzeichnet, daß im Bereich der Sollbruchstelle (21) ein Peel-Streifen (22) zwischen Unterfolie (11) und Oberfolie (12) eingeschweißt ist.

7. Kunststoffpackung nach Anspruch 6, dadurch gekennzeichnet, daß die die Hilfskammer (19) von dem Aufnahmeraum (17) trennende Schweißnaht (16) den Peel-Streifen (22) überdeckt, im Bereich der Sollbruchstelle (21) aber nur einen Teil der Breite des Peel-Streifens (22) erfaßt.

8. Kunststoffpackung nach Anspruch 6 oder 7, dadurch gekennzeichnet, daß die die Hilfskammer (19) von dem Aufnahmeraum (17) trennende Schweißnaht (16) in einem Bereich (24), der sich mit dem Peel-Streifen (22) teilweise überlappt, unterbrochen ist und daß dieser Bereich (24) eine Verbindung zu dem Aufnahmeraum (17) hat.

9. Vorrichtung zur Herstellung einer Kunststoffpackung nach einem der Ansprüche 1 bis 8, mit einer Beutelherstellungsmaschine, die aus einem Folienschlauch oder aus zwei Folien Flachbeutel (10) erzeugt,
**dadurch gekennzeichnet,**
daß eine erste Siegelstation (33) vorgesehen ist, die an den Flachbeuteln eine Querschweißnaht (16) zur Abtrennung einer Hilfskammer (19) von einem Aufnahmeraum (17) erzeugt, daß eine Füllstation (35) zum Einfüllen von Sterilisationsmittel in die offene Hilfskammer (19) vorgesehen ist, und daß hinter der Füllstation (35) eine zweite Siegelstation (36) zum Verschließen der gefüllten Hilfskammer (19) angeordnet ist.

10. Vorrichtung zur Herstellung einer Kunststoffpackung nach einem der Ansprüche 1 bis 8, mit einer Beutelherstellungsmaschine, die aus zwei Folien Muldenbeutel (40) erzeugt, wobei die eine Folie geprägt ist und die andere die Deckfolie bildet und wobei die geprägte Folie (11) mit mindestens zwei Mulden erzeugt wird, von denen eine an einer Einfüllstation (43) mit Flüssigkeit gefüllt wird, bevor die Oberfolie (12) auf die geprägte Unterfolie (11) aufgeschweißt wird,
**dadurch gekennzeichnet,**
daß die geprägte Folie (11) mit einem Aufnahmeraum (17) und zu jedem Aufnahmeraum mit einer kleineren Hilfskammer (19) hergestellt wird, daß die Einfüllstation (43) flüssiges Sterilisationsmittel nur in die Hilfskammer (19) einfüllt und daß eine Beschickungsstation vorgesehen ist, in der medizinische Geräte in jeden Aufnahmeraum (17) eingelegt werden.

11. Vorrichtung nach Anspruch 9 oder 10, dadurch gekennzeichnet, daß eine Vorrichtung zum Zuführen eines Peel-Streifens (22) an derjenigen Siegelstation angeordnet ist, die die Siegelnaht (16) zwischen Aufnahmeraum (17) und Hilfskammer (19) erzeugt.

## Claims

1. A sterile plastic package, comprising a lower sheet (11) and an upper sheet (12) which define a reception chamber (17) containing a medical apparatus, and which are connected at least at one end by means of a transverse welding seam,
**characterized in**
that the reception chamber (17) is adjoined by at least one auxiliary chamber (19) containing a sterilising agent (20), and that a manually breakable rated breaking point (21) is provided between the auxiliary chamber (19) and the reception chamber (17), which can be broken to unblock a passage from the auxiliary chamber (19) into the reception chamber (17) while the sealing with respect to the exterior is maintained.

2. The plastic package according to claim 1, characterized in that the reception chamber (17) contains an absorbing insert (26) for distributing the sterilising agent.

3. The plastic package according to claim 2, characterized in that the absorbing insert (26) is fixed at the upper sheet (12) and/or the lower sheet (11).

4. The plastic package according to claim 2 or 3, characterized in that the absorbing insert (26) is a bag whose opening (27) is located at the end facing away from the auxiliary chamber (19).

5. The plastic package according to claim 4, characterized in that the one edge of the bag opening (27) is connected to the upper sheet (12) and the other one to the lower sheet (11).

6. The plastic package according to one of claims 1 to 5, characterized in that a peel strip (22) is weld between the upper sheet (12) and the lower sheet (11) in the region of the rated breaking point (21).

7. The plastic package according to claim 6, characterized in that the welding seam (16) separating the auxiliary chamber (19) from the reception chamber (17) overlaps the peel strip (22), but covers only a part of the width of the peel strip (22) in the region of the rated breaking point (21).

8. The plastic package according to claim 6 or 7, characterized in that the welding seam (16) separating the auxiliary chamber (19) from the reception chamber (17) is interrupted in a region (24) partially overlapping with the peel strip (22), and that this region (24) is in communication with the reception chamber (17).

9. A device for manufacturing a plastic package according to one of claims 1 to 8, comprising a bag manufacturing machine producing flat bags (10) of a sheet hose or of two sheets,
**characterized in**
that a first sealing station (33) is provided which produces a transverse welding seam (16) for separating an auxiliary chamber (19) from a reception chamber (17) on the flat bags, that a filling station (35) is provided for filling sterilising agent into the open auxiliary chamber (19), and that a second sealing station (36) for closing the filled auxiliary chamber (19) is arranged behind the filling station (35).

10. The device for manufacturing a plastic package according to one of claims 1 to 8, comprising a bag manufacturing machine producing trough bags (40) of two sheets, the one sheet being deformed and the other one forming the cover sheet, and the deformed sheet (11) being produced to have at least two troughs one of which is filled with a liquid at a filling station (43) before the upper sheet (12) is weld onto the deformed lower sheet (11),
**characterized in**
that the deformed sheet (11) is produced to have a reception chamber (17) and a smaller auxiliary chamber (19) for each reception chamber, that the filling station (43) fills liquid sterilising agent only into the auxiliary chamber (19), and that a charging station is provided in which medical apparatus are inserted into each reception chamber (17).

11. The device according to claim 9 or 10, characterized in that a device for feeding a peel strip (22) is arranged at that sealing station which produces the sealing seam (16) between the reception chamber (17) and the auxiliary chamber (19).

## Revendications

1. Emballage stérile en matière plastique avec un film inférieur (11) et un film supérieur (12) qui délimitent un espace de réception (17) contenant un appareil médical et qui sont assemblés à au moins une extrémité par un cordon de soudure transversal, caractérisé en ce que l'espace de réception (17) est suivi d'au moins une chambre auxiliaire (19) qui contient un fluide de stérilisation (20) et qu'entre la chambre auxiliaire (19) et l'espace de réception (17) est prévu un point de rupture obligatoire (21) déchirable manuellement qui peut être cassé pour libérer un passage de la chambre auxiliaire (19) vers l'espace de réception (17), tout en conservant l'étanchéité par rapport à l'extérieur.

2. Emballage en matière plastique suivant la revendication 1, caractérisé en ce que l'espace de réception (17) contient un insert absorbant (26) destiné à répartir le fluide de stérilisation.

3. Emballage en matière plastique suivant la revendication 2, caractérisé en ce que l'insert absorbant (26) est fixé sur le film supérieur (12) et/ou sur le film inférieur (11).

4. Emballage en matière plastique suivant la revendication 2 ou 3, caractérisé en ce que l'insert absorbant (26) est un sachet dont l'ouverture (27) se trouve à l'extrémité éloignée de la chambre auxiliaire (19).

5. Emballage en matière plastique suivant la revendication 4, caractérisé en ce que l'un des bords de l'ouverture de sachet (27) est assemblé au film supérieur (12) et l'autre au film inférieur (11).

6. Emballage en matière plastique suivant l'une des revendications 1 à 5, caractérisé en ce qu'à l'endroit du point de rupture obligatoire (21) est incorporée par soudage une bande arrachable (22) entre le film inférieur (11) et le film supérieur (12).

7. Emballage en matière plastique suivant la revendication 6, caractérisé en ce que le cordon de soudure (16) séparant la chambre auxiliaire (19) de l'espace de réception (17) recouvre la bande arrachable (22), mais ne saisit, à l'endroit du point de rupture obligatoire (21), qu'une partie de la largeur de la bande arrachable (22).

8. Emballage en matière plastique suivant la revendication 6 ou 7, caractérisé en ce que le cordon de soudure (16) séparant la chambre auxiliaire (19) de l'espace de réception (17) est interrompu à un endroit (24) qui est partiellement recouvert par la bande arrachable (22) et que cet endroit (24) possède une communication avec l'espace de réception (17).

9. Dispositif pour la fabrication d'un emballage en matière plastique suivant l'une des revendications 1 à 8, avec une machine de fabrication de sachets qui produit des sachets plats (10) à partir d'un film tubulaire ou à partir de deux films, caractérisé en ce qu'il est prévu un premier poste de scellage (33) qui produit sur les sachets plats un cordon de soudure transversal (16) destiné à séparer une chambre auxiliaire (19) d'un espace de réception (17), qu'il est prévu un poste de remplissage (35) pour le remplissage de fluide de stérilisation dans la chambre auxiliaire (19) ouverte, et que derrière le poste de remplissage (35) est disposé un second poste de scellage (36) pour l'obturation de la chambre auxiliaire (19) remplie.

10. Dispositif pour la fabrication d'un emballage en matière plastique suivant l'une des revendications 1 à 8, avec une machine de fabrication de sachets qui produit des sachets à auges (40) à partir de deux films, l'un des films étant estampé et l'autre constituant le film de recouvrement et le film estampé (11) étant produit avec au moins deux auges dont l'une est remplie de liquide à un poste de remplissage (43) avant que le film supérieur (12) ne soit soudé sur le film inférieur estampé (11), caractérisé en ce que le film estampé (11) est réalisé avec un espace de réception (17) et une chambre auxiliaire (19) plus petite associée à chaque espace de réception, que le poste de remplissage (43) ne remplit du fluide de stérilisation liquide que dans la chambre auxiliaire (19) et qu'il est prévu un poste d'ensachage auquel des appareils médicaux sont déposés dans chaque espace de réception (17).

11. Dispositif suivant la revendication 9 ou 10, caractérisé en ce qu'un dispositif pour l'alimentation d'une bande arrachable (22) est disposé au poste de scellage qui produit le cordon de scellage (16) entre l'espace de réception (17) et la chambre auxiliaire (19).
